# EUROPEAN PATENT APPLICATION

(11) **EP 3 909 540 A1**
(43) Date of publication of application: **17.11.2021**
(21) Application number: 20174624.5
(22) Date of filing: 14.05.2020
(51) Int. Cl.: A61B 90/00, A61B 8/08

(54) **INTERVENTIONAL DEVICE AND A MANUFACTURING METHOD THEREOF**

(71) Applicant: Koninklijke Philips N.V., 5656 AG Eindhoven (NL)
(72) Inventor: WEEKAMP, Johannes Wilhelmus, 5656 AE Eindhoven (NL)
(74) Representative: Philips Intellectual Property & Standards

(57) **Abstract**

An interventional device comprises a shaft and a sensor or actuator element at a distal portion of the shaft. First and second connection wires each extend along a respective side portion of the shaft to the sensor or actuator element. They are positioned without coiling around the shaft.

## Description

### FIELD OF THE INVENTION

The invention relates to interventional devices having a sensor or actuator element at the distal portion of a shaft. In particular, the invention relates to an interventional device with a sensor, such as a needle, guidewire or catheter. The invention also relates to a manufacturing method.

### BACKGROUND OF THE INVENTION

There are many systems which employ sensors or actuators at the tip of an interventional device such as a guidewire, catheter or needle. A sensor may for example be used for measuring flow, pressure, temperature, or function as an ultrasound sensor for example.

One application of interest is for needle tip tracking systems. In such systems, a needle tip is provided with an ultrasound sensor. During an interventional procedure with the needle, ultrasound imaging is performed. The sensor at the needle tip enables the precise position of the needle tip to be represented on the ultrasound image, to assist a physician in performing the interventional procedure.

This needle tip tracking system is for example described in detail in WO2020/030746, WO2020/030642, WO2020/030665 and WO2020/030557. The needle design is for example described in detail in WO2020/030546.

The ultrasound sensor at the needle tip forms a band around the needle. Two connection wires extend along the shaft of the needle to reach the sensor.

These connection wires can fail during bending due to elongation. The current solution to this problem is to coil the wires around the needle shaft, thereby creating a spring like behavior. However, this results in a costly manufacturing process, with each needle having to be manufactured at least in part separately, and it also uses a long length of wire.

This issue applies generally to miniature sensors or actuators at the distal end of elongate shafts. There is a need for an improved electrical connection scheme for connection to such sensors or actuators.

### SUMMARY OF THE INVENTION

According to examples in accordance with a first aspect of the invention, an interventional device is provided, comprising a shaft and an electrical connection element, wherein the electrical connection element comprises:
at least first and second electrical connection wires extending along the shaft, wherein the first and second electrical connection wires comprise an undulating shape along at least a portion of the shaft and wherein each of the first and second electrical connection wires extends along a side portion of the shaft without coiling around the shaft.

In some embodiments the undulating shape may have a different undulation pattern at a first, distal region of the device than the undulation pattern at a second, more proximal region of the device.

The electrical connection element may in some embodiment further comprise a foil rolled around at least a portion of the circumference of the shaft.

Within the disclosure of the invention the terminology of "rolled around at least a portion of the circumference of the shaft" should be broadly construed encompassing also the embodiments wherein the cross-section of the shaft is not circular, in which cases the terminology covers also folding about at least a portion of the cross-sectional perimeter of the shaft.

In some embodiments the foil may comprises metallic material and the first and second electrical connection wires may be electrically insulated from the foil, such that the metallic foil acts as electric shield for the measurement or actuation signal of a sensor. In some embodiment the first and second electric wires may be use for measurement of electrical signal at the distal portion of the interventional device, in which case the exposed distal portion of the distal end or portion of the electrical connection wire cans ense electrical signal from the anatomy, e.g. electrograms or may be used for electrical stimulations of anatomy, e.g heart or nerves.

In some embodiments may comprise a sensor or actuator element at a distal portion of the device, and the at least first and second electrical connection wires extend along the shaft to the sensor or actuator element. The sensor or actuator element may be a pressure sensor, an ultrasound sensor, a temperature sensor, a flow sensor, an electroactive polymer actuator, etc.

In some embodiments the connection element comprises the sensor or actuator element and wherein the sensor or actuator element comprises a layer rolled around at least a portion of the circumference of the shaft.

In some further embodiments the undulating shape may have a different undulation pattern at a first, distal, connection region where the first and second electrical connection wires connect to the sensor or actuator element compared to the undulation pattern along the shaft; and/or the undulating shape may have a different undulation pattern at a second, proximal, connection region where the first and second electrical connection wires connect to proximal circuitry compared to the undulation pattern along the shaft.

In further alternative embodiments the sensor or actuator element may comprise a first terminal at a radially inner surface to which the first connection wire connects and a second terminal at a radially outer surface to which the second connection wire connects.

The interventional device may be one of an interventional needle (diagnostic, biopsy or treatment needle), or any intravascular device such as a sensing or treatment guidewire or catheter.

In some of the embodiments the sensor or actuator element comprises a piezoelectric sensor. The piezoelectric sensor may be one of a pressure sensor, an ultrasound sensor or a combination thereof.

In some embodiments the device may be configured such that allows position tracking of the device within the anatomy of a patient during an ultrasound-guided intervention.

In a second aspect of the invention a method of forming an interventional device is provided, comprising:
providing an electrical connection element by
   providing a foil over a substrate;
   providing double-sided insulating adhesive layer over the foil;
   forming first and second electrical connection wires with an undulating shape;
   providing the first electrical connection wire over the insulating adhesive layer, extending from a proximal portion of the substrate to a distal portion of the substrate;
   providing the second electrical connection wire over the insulating adhesive layer, extending from the proximal portion of the substrate to the distal portion of the substrate; and
rolling the electrical connection element around a shaft such that the foil is rolled around at least a portion of the shaft, and the first and second electrical connection wires each extend along a side portion of the shaft without coiling around the shaft.

In an embodiment of the method, between the steps of providing the first electrical connection wire and providing the second electrical connection wire, a sensor or actuator element layer is provided at a distal portion of the substrate, over the top of the first electrical connection wire; an the second connection wire is provided over the insulating adhesive layer and over the sensor or actuator element layer.

In some embodiments the foil comprises a metallic material, thereby providing shielding to the electrical signals transmitted through the first and second electrical connection wires during operation of the sensor or actuator element.

According to examples in accordance with further aspect of the invention, there is provided a sensor or actuator device comprising:
a shaft;
a sensor or actuator element at or near a distal end of the shaft; and
at least first and second connection wires extending along the shaft to the sensor or actuator element,
wherein the sensor or actuator element comprises a band rolled around an elongate axis of the shaft and the first and second connection wires each extend along a respective side portion of the shaft without coiling around the shaft.

This device avoids the need for the wires to be coiled around the shaft. This enables a reduced wire length as well as a more simple manufacturing process. In particular, multiple sensor or actuator devices can be assembled in parallel. The rolling of the sensor or actuator band around the shaft only requires a single rotation rather than multiple rotations needed to form a coil structure.

The first and second connection wires preferably have an undulating shape along the shaft.

The undulating connection wires provide bending capability, but without needing the wires to be coiled around the shaft.

The undulating shape may have a different undulation pattern at a first, distal, connection region where the first and second connection wires connect to the sensor or actuator element compared to the undulation pattern along the shaft. In this way, the pattern applied to the connection wires may be optimized to provide a best connection to the sensor or actuator terminals.

The undulating shape may have a different undulation pattern at a second, proximal, connection region where the first and second connection wires connect to proximal circuitry compared to the undulation pattern along the shaft. In this way, the pattern applied to the connection wires may be optimized to provide a best connection to the proximal circuitry at which sensor signals are collected and/or processed or at which control signals are generated for an actuator.

The undulation pattern is for example characterized by the amplitude and/or pitch of the undulations. The undulations may be smooth (e.g. following a generally sinusoidal shape) or angular (e.g. following a triangular or square wave shape).

The sensor or actuator element for example comprises a first terminal at a radially inner surface of the band to which the first connection wire connects and a second terminal at a radially outer surface of the band to which the second connection wire connects. Thus, the contact between the sensor or actuator element and the connection wires may be achieved by disposing the sensor or actuator element on top of one connection wire and by disposing another connection wire on top of the sensor or actuator element.

The contact may be made by pressure for example provided with an outer sheath (a heat shrinkable tube) and/or by means of a conductive adhesive or soldering.

The shaft for example comprises a needle, guidewire or catheter. Thus, the invention may be applied to a variety of interventional devices.

The sensor or actuator element may comprise a piezoelectric pressure sensor.

In one set of examples, the sensor or actuator device comprises a needle for implementing needle tip tracking during an ultrasound-guided intervention.

The invention also provides a method forming a sensor or actuator device, comprising:
providing a metal foil over a substrate;
providing a first, double sided, insulating adhesive over the metal foil;
providing a first connection wire over the first insulating adhesive extending from a proximal end of the substrate to a distal end of the substrate;
providing a sensor or actuator element layer at or near the distal end of the substrate, over the top of the first connection wire;
providing a second connection wire over the first insulating adhesive and over the sensor or actuator element layer, the second connection wire also extending from a proximal end of the substrate to a distal end of the substrate; and
rolling the metal foil, the first and second connection wires and sensor or actuator element layer around an elongate axis of a shaft such that the sensor or actuator element layer forms a sensor or actuator element in the form of a band rolled around the elongate axis, with the first and second connection wires extending along a respective side portion of the shaft without coiling around the shaft.

This method enables the substrate to be rolled around the shaft with only one revolution, without any additional coiling of the connection wires. The metal foil acts as an electric shield in the final device.

The method may comprise forming the first and second connection wires with an undulating shape before applying them over the first insulating layer.

The undulation in the connection wires provides improved bending performance.

The first and second connection wires may be formed with an undulating shape having a different undulation pattern at a first, distal, connection region where the first and second connection wires connect to the sensor or actuator element compared to the undulation pattern along the shaft.

The first and second wires may also or instead be formed with an undulating shape having a different undulation pattern at a second, proximal, connection region where the first and second connection wires connect to proximal circuitry compared to the undulation pattern along the shaft.

The method may comprise:
forming a set of sensors or actuators over the substrate, each with respective first and second connection wires with the undulating shape over the first insulating adhesive;
separating the sensors or actuators; and
rolling the separated sensors or actuators around respective shafts at the same time using a same rolling system to form a set of sensor or actuator devices.

In this way, multiple devices may be manufactured by shared processing steps.

The rolling system may comprise a translatable rolling surface for engaging a top surface of the shafts for rolling the shafts as a set. Only one revolution is needed so an array of devices may be rolled side by side.

The shaft of the sensor or actuator device, or of each sensor or actuator device, may be of a needle, guidewire or catheter and the sensor or actuator element of the sensor or actuator device, or of each sensor or actuator device, may comprise a piezoelectric pressure sensor, e.g. such as a PVDF sensor.

The method is for example for forming one or more sensor devices each comprising a needle for implementing needle tip tracking during an ultrasound-guided intervention.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known sensor device in the form of a needle having an integrated distal sensor element;
Figure 2 shows a manufacturing process in accordance with the invention;
Figure 3 shows how the sensors are wrapped around the shafts;
Figure 4 shows the layer structure at different positions along the shaft and the resulting configuration when rolled around the shaft;
Figure 5 shows one resulting sensor device;
Figure 6 shows the sensor at the distal end in the rolled up shape and in the original flat shape;
Figure 7 illustrates the potential problem of straight connection wires;
Figure 8 shows an undulating connection wire to provide bending capability;
Figure 9 shows in schematic form an apparatus for forming the undulating shape, before it is applied to the sensor;
Figure 10 shows an example of one possible configuration for the undulating conductor lines;
Figure 11 shows another example of the conductor line shape in the region of the sensor element; and
Figure 12 shows how external connections may be made to the proximal end.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensor or actuator device, which comprises a shaft and a sensor or actuator element at a distal portion of the shaft, e.g. at or near a distal end of the shaft. The sensor or actuator element comprises a band rolled around an elongate axis of the shaft. First and second connection wires each extend along a respective side portion of the shaft to the sensor or actuator element. They are positioned without coiling around the shaft.

The invention will be described with reference to the example of a sensor element at the end of a shaft. However the same configuration may be applied to an actuator (such as for applying a force or heat).

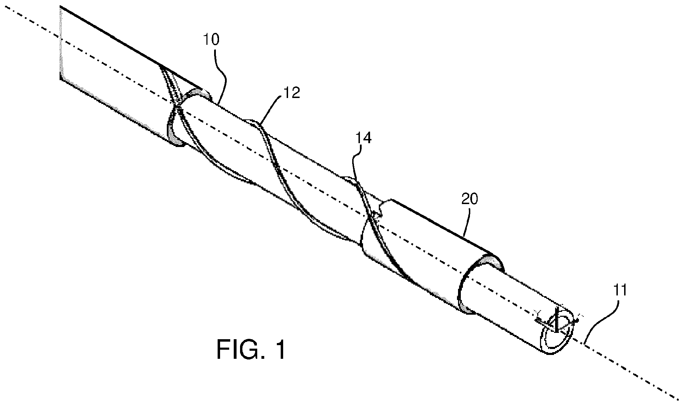

Figure 1 shows a known sensor device in the form of a needle having an integrated distal sensor element.

The sensor device comprises a shaft 10, which in this example is a needle, and a sensor element 20 at or near a distal end of the shaft 10. It is at the distal end or set back from the distal end by a small amount (e.g. by less than 10 times the diameter of the shaft).

First and second connection wires 12, 14 extend along the shaft to the sensor element. The sensor element 20 comprises a band rolled around an elongate axis 11 of the shaft. The first and second connection wires 12, 14 are coiled around the shaft. This enables the wires to tolerate bending of the shaft.

The invention provides an arrangement in which the first and second connection wires are not coiled around the shaft. This simplifies the manufacturing process, and also enables multiple sensor devices to be manufactured simultaneously.

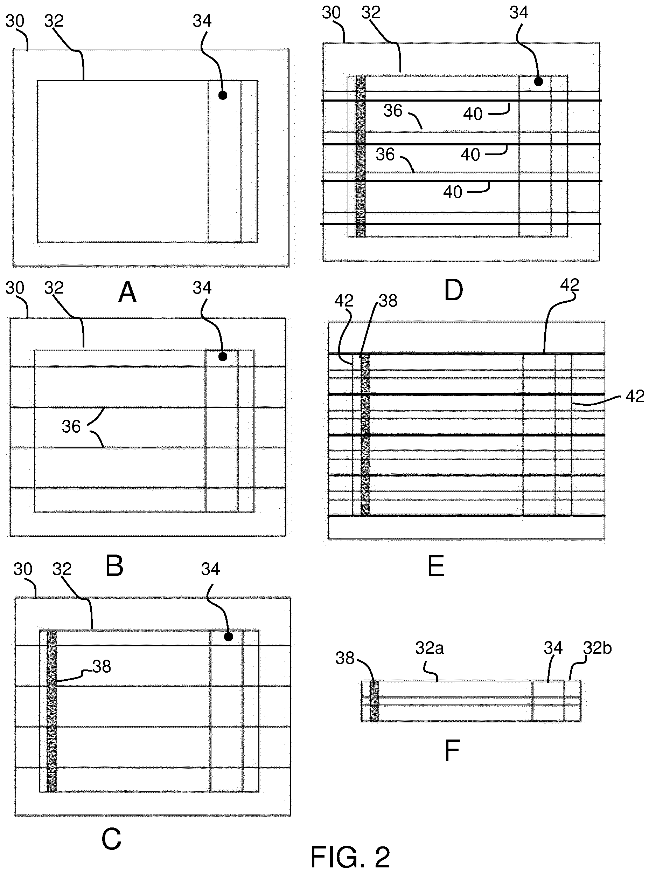

Figure 2 shows the manufacturing process.

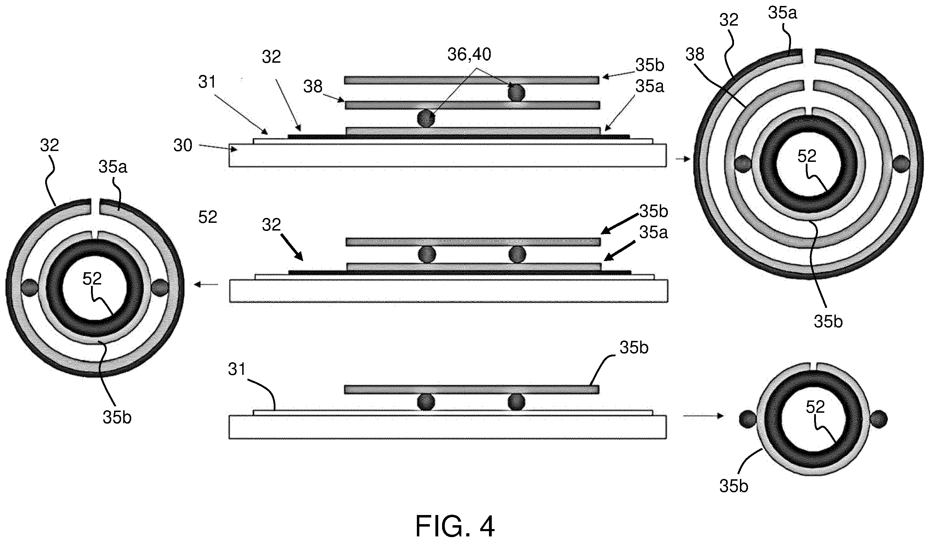

Figure 2A shows a substrate 30 over which a copper foil 32 is provided, with a cut-out region 34. The substrate 30 is a support structure, which does not form part of the final device. The substrate for example comprises a glass plate with a tacky silicone film on top (reference 31 in Figure 4). However, other substrates may be used such as a polymer layer or stack of layers, (e.g. polyethylene terephthalate, PET or Polyimide, PI) or a metal.

The copper foil 32 functions as an electric shielding layer, which when the sensor is formed around a shaft, is around the outside of the sensor element and connection wires (discussed below).

The copper foil 32 is provided with an adhesive coating, optionally a pressure sensitive adhesive coating, on the outer surface (reference 35a in Figure 4). The adhesive acts to bond the next layers together.

Figure 2B shows a first conductive wire 36 provided over the adhesive layer. A set of four such first conductive wires is shown, because the structure of Figure 2 is for forming four sensor devices.

Figure 2C shows a sensor foil 38 formed as a strip perpendicular to the conductive wires 36. The sensor foil 38 comprises in this example a PVDF homopolymer foil strip, with a thin metal contact layer on each side, such as gold. This is a polymer in which a single monomer, specifically, vinyldifluoride, i.e. VDF, is repeated to form the whole polymer. The sensor foil for example has a thickness in the range 8.5 - 9.5 microns and an axial length for example in the range 80 to 120µm. Such a thin layer offers a balance between high flexibility, particularly to allow for wrapping of sensor foil as a band around an elongate shaft, as explained further below, and adequate signal strength and position indication.

The sensor foil 38 is positioned over the first conductive wires, and the bottom surface of the sensor foil (facing the first conductive wires) functions as a first sensor element terminal.

Figure 2D shows a second conductive wire 40 provided over the adhesive layer. A set of four such second conductive wires 40 is shown. The second conductive wires are provided over the sensor foil 38. The top surface of the sensor foil 38 (facing the second conductive wires) functions as a second sensor element terminal.

A further, double sided, insulating adhesive layer is provided over the top (reference 35b in Figure 4). Unlike the first adhesive layer over the copper foil, this layer covers the cut-out region 34 and thereby provides an insulation between the wires and the needle.

The tacky film of the substrate 30 holds the copper foil in position during the application of the wires and sensor element as described above. The tackiness is balanced with the bond strength of the final insulating adhesive layer on top of the final stack. During rolling, the final insulating adhesive layer is able, due to its stronger bond strength, to remove the stack from the tacky film of the substrate.

This then leaves the first and second conductive wires exposed (from below) in the cut-out region 34 but supported by the further, double-sided, insulating adhesive layer.

Figure 2E shows singulation lines 42. The individual sensors (by which is meant the sensor device components but not yet attached to the elongate shaft) are singulated but not physically separated. The singulation for example extends to the glass substrate 30 so that the sensors may be peeled off the glass substrate.

Figure 2F shows the shape of one singulated sensor for illustration purposes.

It has a copper region 32a at the distal end and a copper region 32b at the proximal end. The cut-out region 34 between them defines a contact zone at which connection can be made to the first and second conductive wires, from the outside after the sensor has been rolled around a support shaft.

Each singulated sensor is wrapped around a shaft to form a sensor device. This wrapping creates a sensor element band rolled around the elongate axis of the shaft, but the first and second connection wires each extend along a respective side portion of the shaft without coiling around the shaft. Thus, the connection wires extend generally along one a line at one angular position or a narrow range of angular positions (e.g. in the case of an undulating shape as explained below). The range of angular positions occupied by each connection wire is for example less than 45 degrees, for example less than 25 degrees, for example less than 10 degrees.

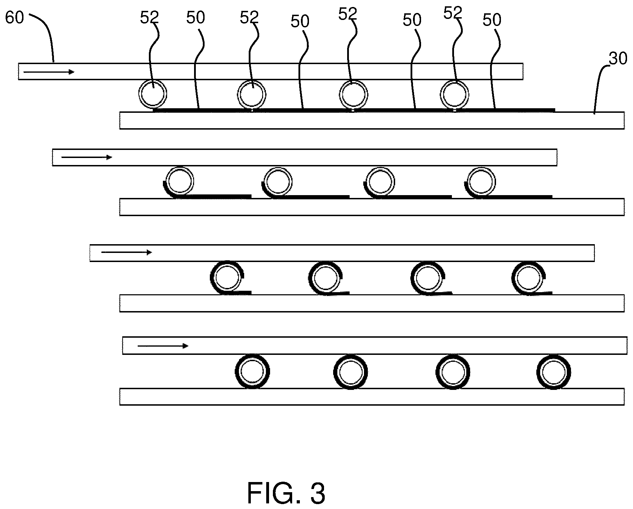

Figure 3 shows how the sensors are wrapped around the shafts, which for example comprise interventional needles, but it may be of guidewire or catheter. Figure 3 shows the set of four sensors 50 viewed in a direction perpendicular to the elongate axis of the needles 52.

The sensors 50 lift off the substrate 30 and are rolled around the needles 52.

Each sensor (i.e. the foil, the first and second connection wires and the sensor layer) is rolled around the elongate axis of a respective shaft such that the sensor element forms a band rolled around the elongate axis, with the first and second connection wires extending along a respective side portion of the shaft without coiling around the shaft.

Figure 3 shows that the separated sensors 50 are each rolled around a respective shaft 52 at the same time using a same rolling system to form a set of sensor devices. The rolling system comprises a translatable rolling surface 60 for engaging a top surface of the shafts for rolling the shafts as a set. The rolling surface 60 is for example another glass plate (similar to the substrate 30) with a tacky silicone film to grip the needle.

Four progressive steps in rolling the sensors around the shafts can be seen in Figure 3. Only one revolution of the shaft is needed to form the sensor element band.

Figure 4 shows in cross section the layer structure at different positions along the shaft and also, again in cross section, the resulting configuration when rolled around the shaft.

The top image shows the distal end at the location of the sensor foil 38.

The tacky film 31 of the substrate, mentioned above, is shown over which the copper foil 32 is provided. The first adhesive layer 35a is shown over the copper foil 32, over which the first conductive wire is mounted. The sensor foil 38 is sandwiched between the first adhesive layer 35a and the second adhesive layer 35b also mentioned above. The cross section in the rolled up configuration shows that the shaft 52 is adhered to the second adhesive layer 35b. The copper foil 32 is released from the tacky layer 31 and thus forms the outermost layer.

The middle image shows the shaft area. It is the same as the distal end but without the sensor foil 38. The two conductive wires are between the two adhesive layers 35a, 35b.

The bottom image shows the proximal end at the location of the cut-out area at which the conductive wires 36, 40 provide external electrical contacts. The absence of the copper foil 32 and the first adhesive layer 35a (which is only present over the copper foil) means the conductive wires are exposed to enable external electrical contact, in a manner which is explained further below. In particular, the wires are for example shaped in this area to enable a pressure electrical contact to be made.

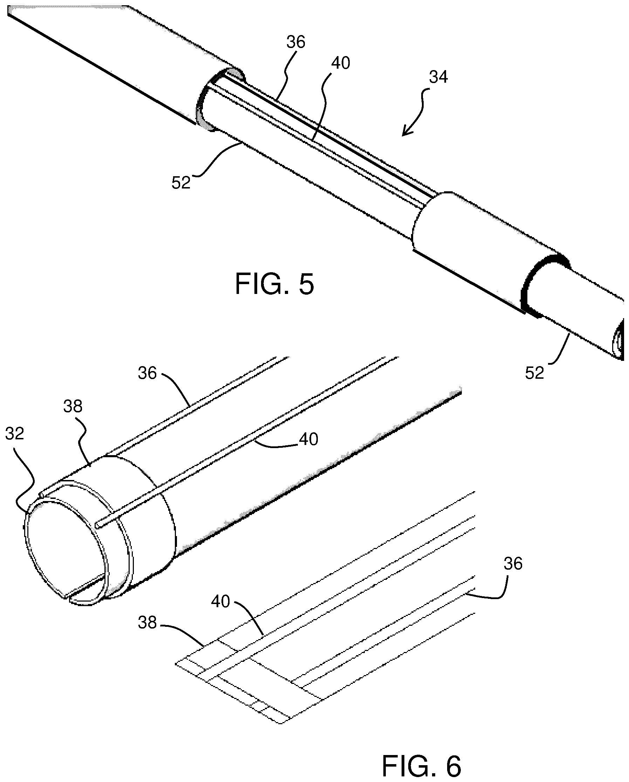

Figure 5 shows one resulting sensor device at the proximal end. The conductive wires 36, 40 are outwardly exposed to enable external contact to be made to the wires, for connection to a processing circuit for receiving and processing the sensor signals, and/or for delivering actuation signals in the case of an actuator. A heat shrink sheath (not shown) is for example provided around the outside.

Figure 6 shows the sensor (i.e. without the shaft) at the distal end (the sensor element end) in the rolled up shape and also in the original flat shape. There is for example a small gap (e.g. 100µm) so that the sensor element band is not closed.

The example above has straight connection wires. This may be appropriate if there is very low strain during bending. This for example depends on the diameter of the shaft. For a guidewire (e.g. 0.35mm diameter) straight wires may be possible. However, if the bending strain is a potential problem, an additional measure may be adopted. This is for example of interest for sensor needles (e.g. 1mm diameter).

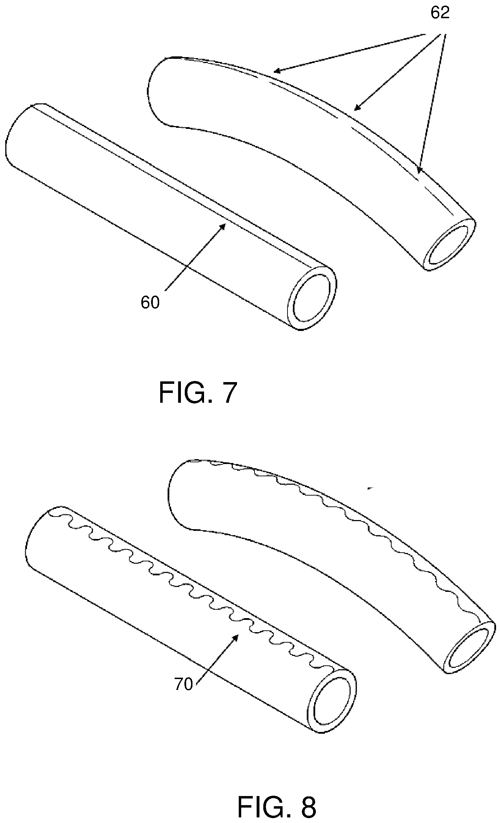

Figure 7 illustrates the potential problem of straight connection wires. The left image shows an unbroken connection wire 60 over a straight shaft, and the right image shows how breaks 62 may form during bending.

Figure 8 shows an undulating connection wire 70 to provide bending capability, but still without needing the wire to be coiled around the shaft. The left image shows an unbroken connection wire 70 over a straight shaft, and the right image shows how breaks are avoided even during bending.

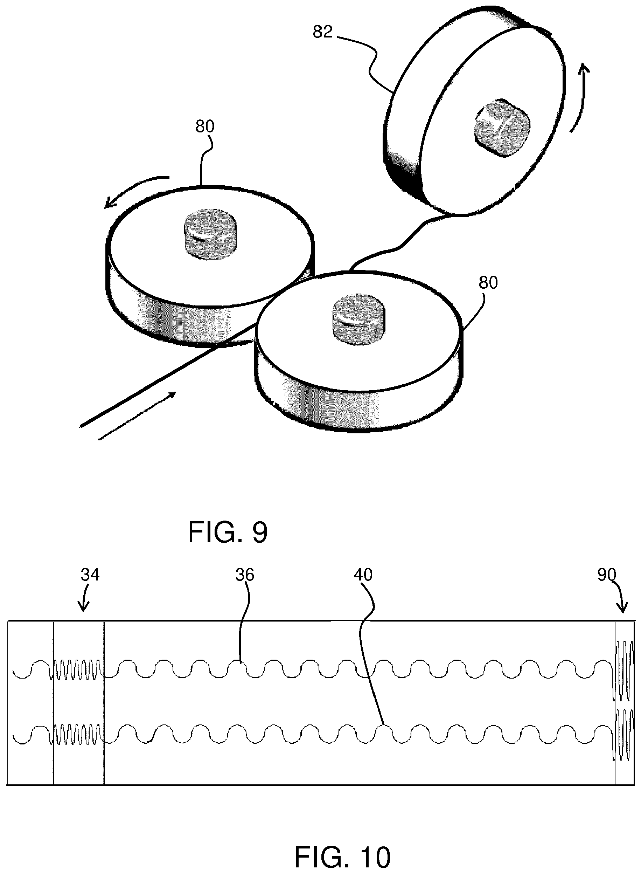

Figure 9 shows in schematic form an apparatus for forming the undulating shape, before it is applied to the sensor.

The apparatus has a pair of rollers 80 between which the wire is fed, and the desired undulating pattern is formed by a guide channel arrangement defined by the mating surfaces e.g. cog teeth of the rollers. The wire is collected on a drum 82. The rollers and drum have correlated rotation speeds.

By translating the drum 82, a set of parallel wires is formed. Part of the processing of the layer structure may therefore be carried out on the drum. By removing a section from the drum, the layer structure may be applied as a patch to the substrate 30 (and its tacky layer 31) during the manufacturing process shown in Figure 2.

The undulation pattern may be adapted by providing a desired guide channel arrangement. A particular selection of the pitch and shape (e.g. amplitude) of the undulations along the length may be used to match the performance to the bending profile. Large undulations result in longer wire length and hence higher resistance but are more resistant to bending.

For example, the undulating shape may have a different undulation pattern at a first, distal, connection region where the first and second connection wires connect to the sensor (compared to the undulation pattern along the shaft). In this way, the pattern applied to the connection wires may be optimized to provide a best connection to the sensor terminals.

The undulating shape may instead or additionally have a different undulation pattern at a second, proximal, connection region where the first and second connection wires connect to proximal circuitry (compared to the undulation pattern along the shaft). In this way, the pattern applied to the connection wires may be optimized to provide a best connection to the proximal circuitry at which the sensor signals are collected and/or processed.

Figure 10 shows an example of one possible configuration for the undulating conductor lines.

At the distal portion, in the region 90 where the conductor lines connect to the sensor, there is an increase in the amplitude of the undulations and a decrease in the pitch so that there is a greater contact area to the sensor.

The electrical contact to the sensor may be based on pressure alone, or else an conductive adhesive may be used. A pressure may be maintained by an applied external sheath, such as a heat shrink sheath.

Similarly, at the cut-out region 34 where the conductor lines connect to the sensor circuitry, there is a decrease in the pitch to create a longer contact area.

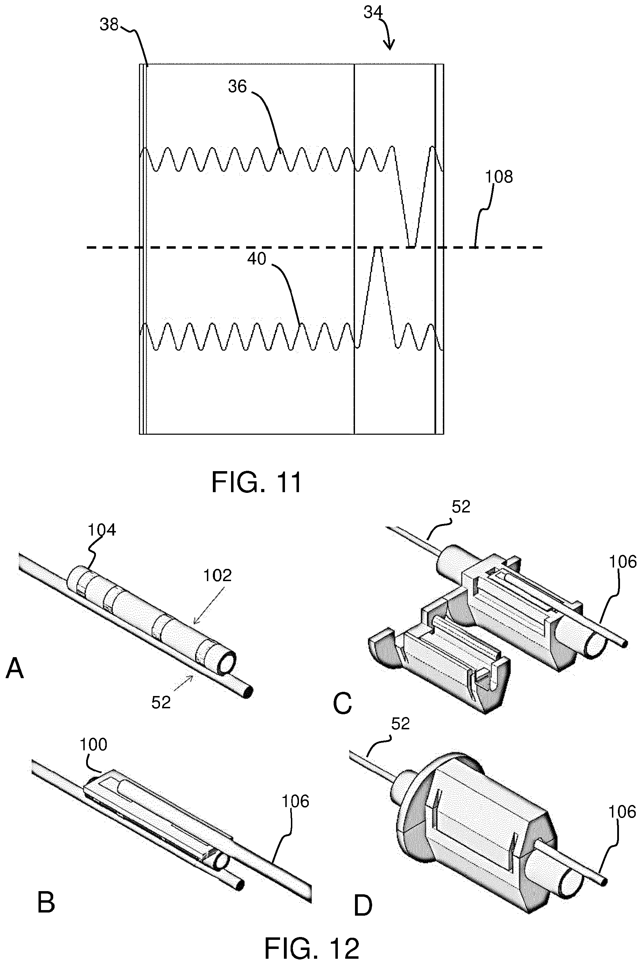

Figure 11 shows another example of the conductor line shape in the cut-out region 34 in order to explain one possible way to connect to the conductive wires in the cut-out region 34. Each conductor line has a single large amplitude and large pitch undulation, and they are at different axial positions so that they form an interleaved pattern.

The known way to provide an interconnect to the exposed wires in a coiled version is based on a pressure contact between the wires and a double-sided PCB.

Figure 12 shows apparatus suitable for this purpose. As shown in Figures 12A and 12B, between the needle 52 and the PCB 100 there is a flexible beam 102 with conductor bands 104. These bands connect the wires, the copper foil shielding and the bare needle to the PCB 100. The contacts between the bands 104 and the needle are all in one line. A coaxial cable 106 connects to the PCB, and this cable leads to the external control circuitry.

The flexible beam may be implemented as an elastomeric electronic connector, for example a Zebra™ connector. This comprises a silicone strip with parallel conducting embedded portions.

Figures 12C and 12D show how the PCB is mounted within a clamp arrangement which fits around the needle and provides a pressure contact between the PCB and the beam and between the beam and the needle.

The shaping of the conductor wires shown in Figure 11 in the cut-out region 34 enables connection to the two conductive wires at different axial positions but along a single line (the dotted line 108 in Figure 11). Thus, the band connections shown in Figure 12 may be used.

The undulations along the shaft are small relative to the shaft diameter, so they may be considered to run straight along the shaft at one general angular position around the shaft, where one general angular position may be understood to mean an angular range of less than 25 degrees, e.g. less than 10 degrees.

The sensor device may include additional electric field shielding layers, not discussed above. There may also be a protective outer tube.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An interventional device comprising a shaft (52) and an electrical connection element, wherein the electrical connection element comprises:
at least first and second electrical connection wires (36, 40) extending along the shaft, wherein the first and second electrical connection wires (36, 40) comprise an undulating shape along at least a portion of the shaft and wherein each of the first and second electrical connection wires extends along a side portion of the shaft without coiling around the shaft.

2. The device of claim 1, wherein the undulating shape has a different undulation pattern at a first, distal region of the device than the undulation pattern at a second, more proximal region of the device.

3. The device of claim 1 or 2, the electrical connection element further comprising a foil rolled around at least a portion of the shaft.

4. The device of any of the preceding claims, wherein the foil comprises metallic material and wherein the first and second electrical connection wires are electrically insulated from the foil.

5. The device of any of the preceding claims, further comprising a sensor or actuator element at a distal portion of the device, and wherein the at least first and second electrical connection wires (36, 40) extend along the shaft to the sensor or actuator element.

6. The device of claim 5, wherein the connection element comprises the sensor or actuator element and wherein the sensor or actuator element (38) comprises a layer rolled around at least a portion of the shaft.

7. The device of claim 5 or 6, wherein:
the undulating shape has a different undulation pattern at a first, distal, connection region where the first and second electrical connection wires connect to the sensor or actuator element compared to the undulation pattern along the shaft; and/or
the undulating shape has a different undulation pattern at a second, proximal, connection region where the first and second electrical connection wires connect to proximal circuitry compared to the undulation pattern along the shaft.

8. The device of 6 or 7, wherein the sensor or actuator element comprises a first terminal at a radially inner surface to which the first connection wire connects and a second terminal at a radially outer surface to which the second connection wire connects.

9. The device of any one of the preceding claims, wherein the device is one of a needle, a guidewire or a catheter.

10. The device of any one of claims 5 to 9, wherein the sensor or actuator element comprises a piezoelectric sensor.

11. The device of claim 10, wherein the piezoelectric sensor is one of a pressure sensor, an ultrasound sensor or a combination thereof.

12. The device of any one of claim 9 to 11, wherein the sensor or actuator element is configured for position tracking during an ultrasound-guided intervention.

13. A method of forming an interventional device, comprising:
providing an electrical connection element by
providing a foil over a substrate;
providing double-sided insulating adhesive layer over the foil;
forming first and second electrical connection wires with an undulating shape;
providing the first electrical connection wire over the insulating adhesive layer, extending from a proximal portion of the substrate to a distal portion of the substrate;
providing the second electrical connection wire over the insulating adhesive layer, extending from the proximal portion of the substrate to the distal portion of the substrate; and
rolling the electrical connection element around a shaft such that the foil is rolled around at least a portion of the shaft, and the first and second electrical connection wires each extend along a side portion of the shaft without coiling around the shaft.

14. The method of claim 13, wherein between the steps of providing the first electrical connection wire and providing the second electrical connection wire, a sensor or actuator element layer is provided at a distal portion of the substrate, over the top of the first electrical connection wire; wherein the second electrical connection wire is provided over the insulating adhesive layer and over the sensor or actuator element layer.

15. The method of claim 13 or 14, wherein the foil comprises a metallic material.
